# FASCICULE DE BREVET EUROPEEN

(11) **EP 2 358 326 B1**
(45) Date de publication et mention de la délivrance du brevet: **06.07.2016**
(21) Numéro de dépôt: 09795412.7
(22) Date de dépôt: 16.12.2009
(51) Int. Cl.: A61H 23/04, A61H 9/00

(54) **DISPOSITIF PULSATILE ET NON INVASIF D'ASSISTANCE CIRCULATOIRE ET HEMODYNAMIQUE**
PULSIERENDE UND NICHT INVASIVE VORRICHTUNG ZUR UNTERSTÜTZUNG VON KREISLAUF UND HÄMODYNAMIK
PULSATILE AND NON-INVASIVE DEVICE FOR CIRCULATORY AND HAEMODYNAMIC ASSISTANCE

(30) Priorité: 16.12.2008 FR 0807077; 23.07.2009 FR 0955168
(43) Date de publication de la demande: 24.08.2011
(73) Titulaire: Nour, Sayed, 92370 Chaville (FR); Chastanier, Pierre, 75008 Paris (FR)
(72) Inventeur: Nour, Sayed, 92370 Chaville (FR); Chastanier, Pierre, 75008 Paris (FR)
(74) Mandataire: Thomas, Nadine
(86) Numéro de dépôt international: PCT/EP2009/067352
(87) Numéro de publication internationale: WO 2010/070018

(56) Documents cités:
- EP-A1- 1 652 557
- EP-A1- 2 033 614
- WO-A1-00/40186
- WO-A1-03/053780
- GB-A- 2 433 034
- US-A1- 2007 282 233
- US-B1- 7 074 200

## Description

La présente invention concerne un nouveau dispositif d'assistance circulatoire.

Elle concerne plus particulièrement un dispositif d'assistance circulatoire et hémodynamique non invasif.

Document US-A-2007/0282233 décrit un appareil de compression ayant une couche intérieure et extérieure avec un élément gonflable disposé dans celui-ci.

Le système circulatoire constitue un circuit hydraulique, fermé, sous pression, tapissé intérieurement par des cellules endothéliales. Cet endothélium est continuellement soumis aux forces tangentielles de cisaillement (shear stress) qui sont indispensables au maintien de sa fonction physiologique : tonus vasculaire grâce à la synthèse de monoxyde d'azote, coagulation du sang, réponse inflammatoire, lutte contre l'athérosclérose, système immunitaire, angiogénèse et apoptose.

Toute altération pathologique de cette fonction endothéliale entraînera un dysfonctionnement du système aux conséquences parfois dramatiques.

Actuellement, il n'existe aucun système d'assistance circulatoire visant à conserver ou à améliorer cette fonction endothéliale.

On connaît les systèmes d'assistance cardiaque qui sont utilisés pour remplacer partiellement ou totalement l'activité cardiaque lors d'opération chirurgicale ou pour retrouver cette activité lorsque le coeur est arrêté ou trop faible. Ces systèmes sont pour la plupart des systèmes invasifs, ils nécessitent soit l'introduction d'un outil dans le corps d'un sujet, cet outil étant ensuite utilisé pour créer des pulsations, soit le prélèvement du sang du sujet et le traitement du sang prélevé dans une machine volumineuse à l'extérieure du corps puis l'injection du sang dans le corps du sujet. Dans tous les cas, les systèmes actuels sont coûteux et compliqués et mettre en oeuvre puisqu'ils nécessitent l'intervention de spécialistes. De plus, ces systèmes ne peuvent être mis en oeuvre que sur des sites dédiés tels que des sites médicaux, sous la surveillance de personnes qualifiées.

Par ailleurs, les systèmes existants présentant une architecture complexe qui rend la fabrication de ces systèmes onéreuse.

En outre, les systèmes actuels permettent de réaliser une intervention générale dans le corps du sujet, en générale au niveau du coeur du sujet et ne permettent pas d'intervenir sur des parties différentes du corps d'un sujet, tel que par exemple les jambes, les mains, le visage ou autre.

Il n'existe donc actuellement aucun système d'assistance circulatoire, non invasif, destiné à la préservation de la fonction endothéliale ou à l'amélioration de cette fonction lorsqu'elle est altérée.

Un but de la présente invention est de pallier les inconvénients précités.

Un autre but de la présente invention est de proposer un dispositif d'assistance circulatoire non invasif destiné à la préservation de la fonction endothéliale ou à l'amélioration de cette fonction lorsqu'elle est altérée.

Encore un autre but de la présente invention est de proposer un dispositif d'assistance circulatoire non invasif, de faible coût, d'architecture simple et simple d'utilisation.

Un autre but de l'invention est de proposer un dispositif d'assistance circulatoire et hémodynamique pouvant être utilisé sur une partie quelconque d'un sujet tel que par exemple les mains, le visage, les jambes, les pieds etc.

Enfin, un autre but de l'invention est de proposer un système d'assistance circulatoire non invasif plus efficace que les systèmes d'assistance cardiaque.

La présente invention permet d'atteindre ces buts par un dispositif d'assistance circulatoire pulsatile non-invasive favorisant la circulation d'un volume sanguin dans le corps d'un sujet, selon la revendication 1.

Le dispositif selon l'invention permet une assistance circulatoire sanguine, de manière non invasive, par l'application de pulsation sur une partie du corps d'un sujet grâce à une structure multicouches.

Le dispositif selon l'invention est simple à utiliser puisqu'il suffit d'appliquer la structure multicouche sur une partie du corps et de créer des pulsations qui se propagent le long de ladite partie du corps grâce aux moyens de pulsations.

Il n'est pas nécessaire que le sujet se déplace sur un site dédié pour l'utilisation du dispositif selon l'invention. En effet, le dispositif selon l'invention peut être utilisé chez le sujet, dans la voiture, lors d'une marche à pied ou une course à pied, lors d'un vol au bord d'un avion, etc.

La structure multicouche peut être appliquée sur une partie quelconque du corps, à l'exception de parties sensible telle que par exemple les parties génitales et oculaires sans les modifications prévues dans les accessoires pulsatiles comme lingerie avec accessoires du sexe masculin ; ou bandage des yeux pulsatile). Ainsi, le sujet peut appliquer le dispositif selon l'invention au niveau d'une partie quelconque du corps telle que par exemple le visage, un bras, une main, un pied, une jambe, le coup, etc... pour réaliser une assistance circulatoire dédiées à cette partie du corps. En effet, le dispositif selon l'invention permet de cibler l'assistance circulatoire sanguine sur une partie du corps en intervenant directement sur cette partie du corps.

Par ailleurs, le dispositif selon l'invention est facile à fabriquer et présente un faible coût de fabrication.

### Théorie

Afin de mieux comprendre les perturbations de l'endothélium dans le système circulatoire, nous allons décrire le phénomène d'interdépendance *« angiogénèse-apoptose »* au regard de la théorie hémodynamique basée sur « le Flux sanguin et le Rythme cardiaque » (Flow and Rate hemodynamic theory) chez l'enfant et l'adulte et découverte par les inventeurs.

Dans le segment artériel, le coeur et les forces péristaltiques poussent le flux sanguin de façon pulsatile avec une pression différentielle (entre systole et diastole) physiologique.

Au contraire, dans les veines et les canaux lymphatiques, sang et lymphe s'écoulent de façon continue sous l'action de forces circulatoires de différentes natures indiquées dans le: mouvements respiratoires (diaphragme, muscles intercostaux), pompe circulatoire musculaire, gravité, pression atmosphérique, récepteurs cutanés, viscosité, intervention du coeur droit (valves, oreillette, ventricule, pression pulmonaire, capacitance veineuse, péricarde).

Le drainage veineux est ainsi directement conditionné par ces forces, qui assurent son retour vers les cavités atrio-ventriculaires droites au moment du remplissage diastolique.

Un bon remplissage du coeur droit (ou précharge), est capital pour un fonctionnement harmonieux de tout le système cardiovasculaire.

L'augmentation de la précharge améliore l'oxygénation musculaire du ventricule droit. Celle-ci dépend plus en effet du remplissage diastolique que des propres réseaux coronariens myocardiques. Cela entraîne une augmentation de sa force contractile qui va améliorer les forces de cisaillement (shear stress) apparaissant dans la circulation pulmonaire. Celles-ci vont provoquer une baisse des résistances vasculaires en raison de l'excrétion du monoxyde d'azote (NO) qu'elles induisent dans l'endothélium pulmonaire et cette baisse des résistances pulmonaires (ou post charge) va améliorer à son tour le débit cardiaque global.

Ceci explique pourquoi les dérivés nitrés si efficaces dans le traitement de l'infarctus du myocarde en cas d'atteinte du ventricule gauche, peuvent au contraire en cas d'ischémie du ventricule droit, risquer d'entraîner le décès du sujet puisque le remplissage de ce ventricule diminuerait en cas d'administration du fait même de l'action vasodilatatrice des nitrites.

Autre exemple évocateur, la position acrobatique assise prise spontanément par un enfant atteint d'une tétralogie de Fallot. Pendant la crise, en raison de l'augmentation des résistances pulmonaires, l'enfant devient bleu. En prenant cette position assise, l'enfant bleu augmente artificiellement les résistances vasculaires du côté gauche ce qui aura pour effet de détourner un plus grand volume pulsatile dans le circuit artériel pulmonaire à travers la communication inter ventriculaire (CIV).

Les forces de cisaillement ainsi augmentées vont forcer l'endothélium pulmonaire à produire plus de NO (monoxyde d'azote) ce qui augmentera immédiatement le flux et le rythme dans l'arbre artériel pulmonaire.

En règle générale toute augmentation des résistances dans un circuit hydraulique entraîne un dysfonctionnement de la pompe d'injection. C'est ce qui explique que l'augmentation des résistances vasculaires (postcharge) du côté gauche entraîne un dysfonctionnement du ventricule gauche dont l'amélioration ne pourra provenir que de la baisse de cette postcharge (action vasodilatatrice des nitrites en cas d'infarctus gauche).

Le coeur droit dans la crise de Fallot sollicite donc paradoxalement l'augmentation de la postcharge à gauche pour faire baisser la sienne ! Cela signifie qu'il n'hésite pas à mettre provisoirement en péril le coeur gauche pour améliorer sa propre hémodynamique et ensuite seulement améliorer à nouveau celle du coeur gauche (Tableau 1 : *The Bossy Right Heart) !*

À l'heure actuelle, en cas de défiance du ventricule droit, le schéma thérapeutique habituel consiste à :
a) augmenter le volume sanguin par des perfusions intraveineuses.
b) augmenter la fréquence cardiaque (atrial kick), par des chronotropes ou par un pacemaker (entraînement électrique).

C'est dans les deux cas une augmentation des forces de cisaillement (volume et rythme) obtenue par des méthodes non-physiologiques et non dénuées d'effets secondaires.

**Tableau 1 : Domination du Coeur droit sur le Coeur gauche à travers les résistances pulmonaires :**

| | **Coeur Droit** | **Coeur Gauche** |
|---|---|---|
| **Résistances Systémiques Basses** | *Mauvaise* hémodynamique ¹ | *Bonne* hémodynamique |
| **Résistances Systémiques Élevées** | *Bonne* hémodynamique ² | *Mauvaise* hémodynamique |
| **Résistances Pulmonaires Basses** | *Bonne* hémodynamique | *Bonne* hémodynamique |
| **Résistances Pulmonaires Élevées** | *Mauvaise* hémodynamique | *Mauvaise* hémodynamique |

| | | |
|---|---|---|
| 1= Nitrites et Infarctus du Ventricule droit. 2 = Crise de Fallot. | | |

Nous considérons, contrairement au concept généralement admis, que le coeur droit domine le développement et l'hémodynamique du coeur gauche depuis la vie anténatale. Au cours de la vie intra-utérine en effet, bien que le ventricule droit (VD) reçoive 2/3 du volume sanguin corporel, les parois des veines et du ventricule droit gardent un remodelage bas par rapport aux artères systémiques en raison de l'existence de shunts physiologiques (ductus venosus, canal artériel, foramen ovale).

Après la naissance et en raison de la fermeture des shunts physiologiques chaque ventricule reçoit le même volume sanguin qui sera éjecté avec la même fréquence. Soumis à des conditions rhéologiques identiques le ventricule droit ne représente pourtant que 1/6 de la masse myocardique du ventricule gauche (VG).

Ceci s'explique en raison de deux facteurs majeurs :
A. Cardiaque : En dehors des caractéristiques déjà décrites dans la littérature (morphologie sphérique de la cavité ventriculaire droite, distribution des fibres, axe de contractilité, etc.) nous insistons sur le rôle majeur joué par le muscle trabéculaire qui tapisse l'intérieur de la face antérieure de l'oreillette droite et la plus grande partie de la cavité ventriculaire (hormis le septum et l'infundibulum). Nous avons souligné l'importance de ce concept dans notre nouvelle classification du coeur droit divisé en cinq zones.
B. Extracardiaque : Sous le contrôle des forces accessoires détaillées ci-après.

Nous considérons en particulier que la pompe respiratoire possède un effet direct sur le contrôle physiologique du système circulatoire.

### Forces de cisaillement physiologiques extravasculaires influençant la fonction endothéliale

### A. La pompe respiratoire « Maître» du système cardio-endothélial :

A la manière d'un « Accordéon » les mouvements d'inflation / déflation des poumons créent une force de cisaillement externe sur les vaisseaux pulmonaires. Leurs effets impressionnants démarrent après la naissance à la première inspiration, qui entraîne une baisse immédiate des résistances pulmonaires et déclenche la fermeture des shunts en commençant par la valvule du foramen ovale puis en continuant, en quelques jours, par le ductus venosus et le canal artériel.

L'échec des anastomoses cavo-pulmonaires chez les nourrissons de moins de 2 ans se rapporte d'ailleurs selon notre expérience clinique à l'incapacité de la pompe respiratoire, en raison du développement insuffisant des muscles de la cage thoracique, d'assurer par des forces de cisaillement suffisantes le drainage veineux nécessaire.

### B. Fluctuations/Propagations des ondes pulsatiles externes provoquant des réactions endothéliales :

De même, une différence entre tumeurs malignes et tumeurs bénignes pourrait provenir de la présence ou non d'une capsule qui joue un rôle protecteur contre la propagation des ondes pulsatiles provenant des organes voisins. Ceci peut expliquer le pronostic plus péjoratif des cancers des organes mobiles (estomac, poumons) ainsi que des tumeurs d'organes richement vascularisés comme le cerveau, comparé à celui des cancers d'organes plus fixes comme la thyroïde ou la prostate.

### Nous l'expliquons par le fait que toute stimulation externe de la fonction endothéliale accélère l'anqiogénèse donc ici, la croissance tumorale

Autre exemple : les malformations congénitales sont le plus souvent associées à une diminution pendant les premiers mois de la grossesse, du liquide amniotique qui isole le foetus des ondes pulsatiles propagées par les organes maternels voisins.

{Centre Hospitalo-Universitaire, Strasbourg, France : C. Stoll; et al. Study of 224 Cases of Oligohydramnios and Congenital Malformations in a Series of 225,669 Consecutive Births. Community Genet 1998; 1:71-77}.

Le principe sur lequel se base la présente invention permet de diviser le coeur droit en cinq zones morphologiques (masse ventriculaire et épaisseur des parois vasculaires) selon la réponse aux forces de cisaillement effectuées sur les parois endothéliales. Sayed Nour et al. The Forgotten Driving Forces In Right Heart Failure. Asiatic Ann Cardiovasc Thorac Surg. (In Persse).

Ces cinq zones sont les suivantes:
- **Zone 1:** représentée par le système veineux, faiblement remodelée en raison de l'absence de forces rythmiques. Le flux sanguin s'écoulant en basse pression dans cette zone est sous l'influence des forces circulatoires accessoires (Tableau1).
- **Zone 2:** représentée par la cavité atrio-ventriculaire, où le flux sanguin de retour veineux commence à s'animer (rythme et pression) ce qui entraîne un remodelage modéré. Le muscle trabéculaire joue ici un rôle de frein naturel atténuant les forces des cisaillements exercées sur la paroi ce qui permet à celle-ci de se contenter d'une épaisseur de 1/6 de celle du ventricule gauche (qui ne possède pas une importante zone trabéculaire). Dans cette zone l'hémodynamique dépend du remplissage diastolique (la précharge) indispensable pour nourrir le muscle ventriculaire droit surtout dans sa partie trabéculée.
- **Zone 3:** C'est le septum inter ventriculaire qui garde une morphologie normale à gauche comme à droite, liée à sa vascularisation par les artères inter septales. L'hémodynamique de cette zone dépend indirectement de celle du ventricule gauche (vascularisation commune) et directement des forces de cisaillement s'exerçant à droite afin de baisser la postcharge pulmonaire (ce qui entraînera une amélioration hémodynamique consécutive à gauche).
- **Zone 4:** représentée par l'infundibulum avec un remodelage très élevé conséquence de l'importance des forces de cisaillement nées de la première artère inter septale. L'hémodynamique de cette zone dépend donc des forces de cisaillement (volume et rythme) et de la surcharge de pression de la 1^{er} artère inter septale.
- **Zone 5:** représentée par l'arbre pulmonaire artériel, zone peu remaniée, avec un pourcentage diamètre-épaisseur de paroi quasi identique à celui des grandes veines. L'hémodynamique de cette zone dépend des résistances vasculaires (baisse de la postcharge), elles-mêmes liées aux forces de cisaillement (surtout le rythme car l'arbre arrive à baisser sa pression artérielle, grâce à sa compliance, bien qu'il reçoive le même volume sanguin que l'aorte.

Les perturbations des forces accessoires peuvent entraîner un dysfonctionnement endothélial. Citons quelques exemples, selon notre classification précédente, afin de comprendre le phénomène :
En **Zone 1,** fortement dépendante de ces forces accessoires, on peut constater que les altérations de celles-ci provoquent des troubles cardiovasculaires et circulatoires presque identiques chez les astronautes et chez les plongeurs professionnels. Malgré la grande différence de pression constatée dans ces deux cas (absente chez les astronautes, très forte chez les plongeurs) les troubles observés sont liés à la défaillance de la pompe de drainage veineux (capacitance veineuse élevée par manque de gravité dans l'espace, et par écrasement dans l'eau).

Il en est de même pour le développement précoce des rides chez les plongeurs, et l'oedème facial sévère en haute altitude (Siobhan Gill, Neil M. Walker, Severe facial oedema at high altitude, Journal of Travel Medicine Volume 200815, Issue 2, Pages130 - 132, International Society of Travel Medicine}.

En dehors de ces conditions extrêmes, l'oedème du visage autour des yeux (paupières gonflées) se manifeste plus le matin après une longue nuit de sommeil, (avec parfois des maux de tête) pour disparaître progressivement avec la reprise des activités.

Cette congestion lymphatique démontre l'effet de la diminution de la gravité sur le retour veineux de la face, entraînant une accumulation de produits toxiques (syndrome inflammatoire, radicaux libres, ralentissement de la circulation caverneuse).

Chez l'enfant, pourtant, malgré une vascularisation et une surface de la face plus importantes que chez l'adulte, l'effet de la gravité pendant les longues périodes de sommeil reste minime.

La formule de Parkland connue sous le nom de « loi de 9's » appliquée chez les grands brûlés prouve l'importance de la surface corporelle de la tête comparé au reste du corps (18%) chez les enfants contre (9%) adultes.

Un bon sommeil en effet favorise l'anabolisme (réparation et régénération) du processus angiogénèse - apoptose qui dépend des forces de cisaillement complétées par un bon drainage veineux. Or, l'enfant ou le nouveau-né ont un rythme cardiaque très élevé parfois double de celui de l'adulte (même pendant le sommeil).

Ces forces de cisaillement sont donc primordiales pour permettre l'accélération naturelle de la croissance. Avec un tel flux, un tel rythme et une telle surface faciale l'enfant garde toujours un visage lisse, sans le moindre signe de tuméfaction, avec une peau satinée même après de très longues périodes de décubitus dorsal.

La différence morphologique entre adulte et enfant joue donc un rôle important dans l'explication de ce phénomène.

De plus, afin d'assurer un bon drainage veineux évitant les effets secondaires causés par la gravité pendant le sommeil, deux autres éléments viennent compléter l'action des forces accessoires de la circulation :
- les cris qui représentent un formidable exercice de pompe musculaire au niveau du visage empêchant la stase veineuse.
- un cou quasi absent (web neck) rendant le drainage veineux encore plus dépendant de la pompe respiratoire.

Les effets hémodynamiques dans les autres zones, Zone 2 à Zone 4, sont également troublés par une diminution des retours veineux en Zone 1. Les effets cardio-pathogéniques directs (ischémie du myocarde ou malformation cardiaque) peuvent donc provoquer des troubles hémodynamiques majeurs.

Maintenir en Zone 5, qui est une zone clé, une bonne hémodynamique est la condition d'un bon fonctionnement global du système circulatoire. Des résistances élevées en Zone 5 (postcharge) peuvent provoquer des troubles hémodynamiques rétrogrades avec une dépression hémodynamique systémique. Les syndromes d'hypertension pulmonaire aigue ou chronique dépendent du niveau d'excrétion du monoxyde d'azote et du remodelage vasculaire, autrement dit des forces de cisaillement.

En résumé, alors que dans les conditions physiologiques les forces accessoires de la circulation assurent le drainage veineux et lymphatique, un dysfonctionnement endothélial entraînera des stases veineuse et lymphatique responsables du des troubles circulatoire et hémodynamique : signes de fatigues (troubles de système immune et réponse inflammatoire, vieillissement précoce (troubles de l'angiogénèse-apoptose). De ces constatations découlent l'invention de nouveaux dispositifs d'assistance circulatoire. → à prévoir après le dernier document

### RESUME

1- En cas de défiance de la pompe cardiaque :
   Nous conseillons l'application de fréquences de cisaillement plus rapides que le rythme cardiaque en Zone 5 (artère pulmonaire), permettant de créer un vortex près de la paroi artérielle (écoulement rotationnel avec dissipation d'énergie sous l'effet de la viscosité - principe de Bernoulli) sans augmenter la pression (Newton) afin d'éviter la compliance ou distensibilité de l'artère pulmonaire (se terminant par un syndrome d'Eisenmenger) ou de perturber à long terme la disposition monocellulaire de l'endothélium alvéolaire.
   Au contraire si nous utilisons un système de pulsations externes jouant à distance sur la Zone 1 comme notre modèle de pantalon pulsatile, les fréquences de cisaillement doivent être impérativement plus lentes que le rythme cardiaque (pas plus que 50%) afin de ne pas suralimenter par ces compressions externes, qui augmentent les forces de cisaillement, un circuit ventriculaire et pulmonaire déjà surchargé.
2- En cas de risques circulatoires sur un coeur normal (astronautes, plongeurs) les fréquences utilisées pour la combinaison pulsatile doivent être synchronisées sur la phase diastolique, sauf en cas de troubles respiratoires ou de tachycardie.
   Sur la circulation périphérique (Masques, chaussons, bottes ...) elles peuvent-être plus rapides que le rythme cardiaque sans aucun danger.
3 - Finalement en cas de défaillance cardiaque, nous adaptons les forces de cisaillement générées par nos dispositifs pulsatiles aux besoins du système endothélial selon la partie du circuit concernée.

### Applications

Les inventeurs ont découvert que le dispositif selon l'invention peut être utilisé pour une multitude d'applications reliées toute à la fonction endothéliale.

Les inventeurs ont découvert que le vieillissement, par exemple, est en réalité la conséquence d'une perturbation de la fonction endothéliale mettant en oeuvre un processus d'interdépendance *angiogénèse-apoptose* dont les signes précoces comme les rides ou les cheveux gris apparaissent justement dans une des parties les plus vascularisées de notre corps (visage et tête). Ce vieillissement est un phénomène naturel lié au ralentissement progressif du processus de remplacement par l'angiogénèse des cellules mortes (mort cellulaire programmée ou apoptose) mais il est singulièrement accéléré chaque fois que des facteurs secondaires (infection, syndrome ischémique, traumatismes, radiations X ou UV, syndrome dégénératif) affectent la fonction endothéliale (syndrome inflammatoire, système immunitaire, vasoconstriction).

Selon une particularité avantageuse du dispositif selon l'invention, les moyens de pulsation peuvent être adaptés pour générer des pulsations à un rythme fonction de :
- données relatives au rythme cardiaque,
- données relatives au rythme de respiration,
- données relatives à l'état de santé du sujet, et/ou
- données relatives à la partie à laquelle est appliquée la structure multicouche.

Le dispositif selon l'invention peut comprendre des moyens pour mesurer le rythme cardiaque et des moyens pour mesure le rythme de respiration.

Le dispositif selon l'invention peut comprendre des moyens pour modifier, régler et choisir le rythme de pulsations générées par les moyens de pulsations.

Le rythme de pulsations peut être déterminé en fonction des besoins du sujet. Les expérimentations animales menées par les inventeurs permettent de différencier les cas suivants pour la régulation du rythme de pulsation en fonction de l'état du sujet et en fonction de la zone du corps sur laquelle est appliquée la structure multicouche du dispositif selon l'invention :
- en cas de **défiance de la pompe cardiaque** chez le sujet :
   o dans la Zone 5, zone de l'artère pulmonaire, la théorie décrite dans le précédent brevet « MICROTH » et publication qui a été confirmée par les expérimentations montrent qu'il est nécessaire d'appliquer une fréquence de cisaillement plus rapide que le rythme cardiaque, permettant de créer un vortex près de la paroi artérielle (écoulement rotationnel avec dissipation d'énergie sous l'effet de la viscosité - principe de Bernoulli) sans augmenter la pression (Newton) afin d'éviter la compliance ou distensibilité de l'artère pulmonaire (se terminant par un syndrome d'Eisenmenger) ou de perturber à long terme la disposition monocellulaire de l'endothélium alvéolaire.
   o si le dispositif est utilisée pour agir sur la Zone 1, c'est dire le système veineux, par exemple sous la forme d'un pantalon pulsatile, la fréquence de cisaillement doit être impérativement plus lente que le rythme cardiaque, aux alentours de 50% du rythme cardiaque, afin de ne pas suralimenter par des compressions externes, qui augmentent les forces de cisaillement, un circuit ventriculaire et pulmonaire déjà surchargé.
- en cas de **troubles circulatoires sur un coeur normal,** par exemple pour les, syndrome-X ; les diabétiques ou les hypertendus (sans effets secondiares cardiaque) ; effets secondaires de ménopause ; astronautes ou les plongeurs :
   o la fréquence utilisée dans la Zone 5, doit être synchronisée sur la phase diastolique, sauf en cas de troubles respiratoires ou de tachycardie.
   o sur les autres zone périphériques, masques, chaussons ou bottes pulsatile, la fréquence peut être plus rapide que le rythme cardiaque sans aucun danger.
- Chez les **sujets normaux** sans maladie cardiaque ni circulatoire, par exemple chez les sportifs : même si les athlètes sont capables de s'adopter avec leur retour veineux (selon la loi de Frank-Starling du Coeur) : 1) c'est toujours recommandé de surveiller la synchronisation diastolique c'est l'environnent nous permit, comme dans les sale de Gym ou massage. 2) chez les utilisateur comme pendant les réchauffement avant les matches ou jogging, un control et visite régulier par de spécialistes cardiaques afin de mettre le règles à suivre par le sportif.

En résumé il faudra impérativement garder une contacte médicale régulièrement pour faire le bon choix au fûr et à mesure de l'amélioration hémodynamique: en cas de défaillance cardiaque, la fréquence doit être adaptée aux besoins du système endothélial selon la zone concernée sans synchronisation, contrairement aux autres applications sans maladie cardiaques.

Dans un mode de réalisation particulier, le dispositif selon l'invention peut comprendre un module comprenant d'une part des moyens sélection permettant à un utilisateur de sélectionner des données relatives à son état physique, tel que l'âge, la taille, le poids, l'état de son coeur, etc... des moyens de mesure du rythme cardiaque et du rythme respiratoire, et des moyens de calcul du rythme des pulsations en fonction d'une ou plusieurs de ces données selon une ou plusieurs relations prédéterminées.

Avantageusement, la couche interne de la structure multicouche peut comporter, sur au moins une partie, une cavité entre une paroi microporeuse destinée à être mise en contact avec la peau du sujet et une paroi étanche du côté de la couche externe, ladite cavité étant aménagée pour recevoir, stocker et/ou conduire un produit destiné à être appliquée sur la peau dudit sujet au travers de ladite paroi microporeuse.

Ainsi, le dispositif selon l'invention permet d'appliquer un ou plusieurs produits biologiques ou cosmétologiques et permet leur diffusion sur la partie du corps sous-jacente de façon homogène.

Dans ce cas, la structure multicouche du dispositif selon l'invention peut comporter une ouverture pour remplir la cavité avec un produit. Cette ouverture peut lors de l'utilisation du dispositif selon l'invention soit être connectée à un réservoir de produit par un moyen de connexion, soit être fermée de manière étanche par des moyens d'obturation ladite cavité étant pré rempli et servant alors de réservoir de produit également.

Selon un mode de réalisation particulier, la structure multicouche peut comprendre une ouverture d'admission du fluide de pulsation provenant des moyens de pulsation, entre les couches interne est externe et un fluide gélatineux et/ou granuleux entre lesdites couches externe et interne réalisant la propagation progressive de chacune des pulsation dans le sens du retour veineux entre le long de la partie du corps sur laquelle la structure multicouche est appliquée.

Le fluide gélatineux ou granuleux peut selon une particularité de l'invention être est contenu dans une couche intermédiaire entre la couche externe et la couche interne.

Selon une première version des moyens de pulsation, les moyens de pulsation peuvent comprendre :
- un réservoir pneumatique,
- un moyen de compression dudit réservoir pneumatique de manière rythmique, et
- un connecteur étanche reliant ledit réservoir pneumatique à la structure multicouche flexible.

Le moyen de compression peut être mécanique, et actionné par le sujet lui-même ou par une source d'énergie externe portable ou non.

Selon une deuxième version des moyens de pulsation, les moyens de pulsations peuvent comprendre :
- un réservoir pneumatique pré-rempli, et
- un connecteur étanche reliant ledit réservoir pneumatique à la structure multicouche flexible ;
l'ensemble réservoir, connecteur et structure multicouche constituant un circuit fermé pour le fluide de pulsation, et
ledit réservoir pneumatique étant disposé de sorte qu'il est compressé et décompressé par une force exercée par ledit sujet.

Cette force exercée peut être exercée par le sujet lui-même par une fermeture/ouverture de poignets lorsque le réservoir est disposé dans la main de l'utilisateur.

La force peut aussi être exercée par une pression/dépression créées par au moins une chaussure du sujet frappant une surface lors d'une marche ou d'une course par exemple. Dans ce cas, le réservoir pneumatique pré-rempli est disposé sous ou dans une chaussure du sujet de sorte que lorsque le sujet exerce une pression en appuyant sur son pied, le réservoir est vidé du fluide qu'il contient et le fluide est injecté dans la structure multicouche en générant une pulsation et lorsque le sujet relâche la pression sur son pied, par exemple en le levant, le fluide injecté dans la structure multicouche est rappelé dans le réservoir pneumatique.

Dans un mode de réalisant particulier, le réservoir pneumatique, le connecteur et la structure multicouche flexible peuvent constituer un ensemble monobloc, lorsque par exemple, la structure multicouche compose une botte destinée à être chaussée par le sujet.

La structure multicouche flexible peut en outre comprendre une cagoule destinée à être disposée sur au moins une partie du visage du sujet.

En outre, la structure multicouche flexible peut comprendre un pantalon.

Avantageusement, la structure multicouche flexible peut comprendre une veste.

La structure multicouche flexible peut en outre comprendre comprend un ou plusieurs gants ou une partie d'un gant destiné(e)(s) à être appliqué sur au moins une partie de la main et/ou du poigné du sujet.

Par ailleurs, la structure multicouche flexible peut comprendre une botte une chaussure, ou une chaussette. Dans ce dernier cas, les moyens de pulsation peuvent être intégrés dans la semelle de la botte, chaussure ou chaussette, de sorte que les pulsations sont créées par la marche ou la course du sujet, les pressions créées par l'appui du pied sur un sol provoquant le gonflement progressif de la structure multicouche, et la levée du pied provoquant un dégonflement, progressif ou non de la structure multicouche.

La structure multicouche peut comprendre :
- des éléments de lingeries pulsatiles tel que corsets, bas, ... etc utilisés par exemple pour le traitement de la cellulite, ou des troubles des rapports sexuels chez l'homme et la femme,
- des anneaux pulsatiles applicables sur les membres inférieurs ou supérieurs chez les diabètes et les sujets hypertensifs ;
- des accessoires orbitales, par exemple en forme de bandage pour les yeux pour le traitement des rides.

Selon un autre aspect de l'invention, il est proposé un ensemble d'assistance circulatoire pulsatile non-invasive couvrant plusieurs parties du corps d'un sujet, ledit ensemble comportant au moins plusieurs dispositifs selon l'une quelconque des revendications précédentes pour chacune des dites parties, chacun des dispositifs étant indépendant.

Selon encore un autre aspect de l'invention il est proposé un ensemble d'assistance circulatoire pulsatile non-invasive couvrant plusieurs parties du corps d'un sujet, comprenant :
- pour chacune desdites parties, une structure multicouche flexible destinée à être appliquée sur ladite partie du corps dudit sujet, ladite structure comportant une couche interne flexible du côté du corps dudit sujet et une couche externe plus rigide
- des moyens de pulsation communs auxdites structures pulsatiles, lesdites moyens de pulsation étant reliés de manière étanche à chacune desdites structures multicouche de sorte,
caractérisé en ce que lesdits moyens de pulsation sont adaptés à créer des pulsations entre lesdites couches internes et externes de chacune desdites structures par l'intermédiaire d'un fluide, dit de pulsation, chacune desdites pulsations au niveau de chacune desdites parties se propageant progressivement dans le sens du retour veineux dans ladite partie du corps dudit sujet lorsque lesdites structure sont disposées sur le corps dudit sujet.

D'autres avantages et caractéristiques de l'invention apparaîtront à l'examen de la description détaillée d'un mode de mise en oeuvre nullement limitatif, et des dessins annexés sur lesquels :
- la figure 1 est une représentation schématique d'un exemple de structure multicouche mise en oeuvre dans le dispositif selon l'invention ;
- la figure 2 est une représentation schématique d'une console permettant de créer des pulsations dans la structure multicouche dans la structure multicouche de la figure1 ;
- la figure 3 est une représentation schématique d'un module de compression permettant de créer des pulsations en combinaison avec la console de la figure 2 ;
- la figure 4 est une représentation schématique d'un module de détermination d'un rythme de pulsation ;
- la figure 5 est une représentation schématique d'une cagoule pulsatile selon l'invention ; et
- la figure 6 est une représentation schématique d'un pantalon pulsatile selon l'invention.

Le dispositif selon l'invention produit des mouvements rythmiques harmonieux et progressifs sur tout ou partie de l'organisme en ramenant le sang des extrémités vers le coeur au moment de la diastole. Il produit des forces de compression non agressives et destinées à réduire la capacitance veino-lymphatique stagnant le plus souvent dans les tissus sous-cutanés, la circulation hépatique- splénique ou la face.

Nous allons maintenant décrire plusieurs exemples de dispositif selon l'invention.

Dans tous les exemples qui vont être décrits,
- les axes de propagation des « vagues pulsatiles » sont déterminés de façon à conserver les directions naturelles et physiologiques des drainages veineux et lymphatiques.
- les forces pulsatiles, peuvent-être produites par un système pneumatique, électronique, hydraulique ou même autonome utilisant la propre force du sujet, et
- les parties dorsales normalement non gonflables pour protéger le rachis des traumatismes, peuvent être modifiées dans les versions destinées aux massages corporels tout en conservant leurs sécurités essentielles.

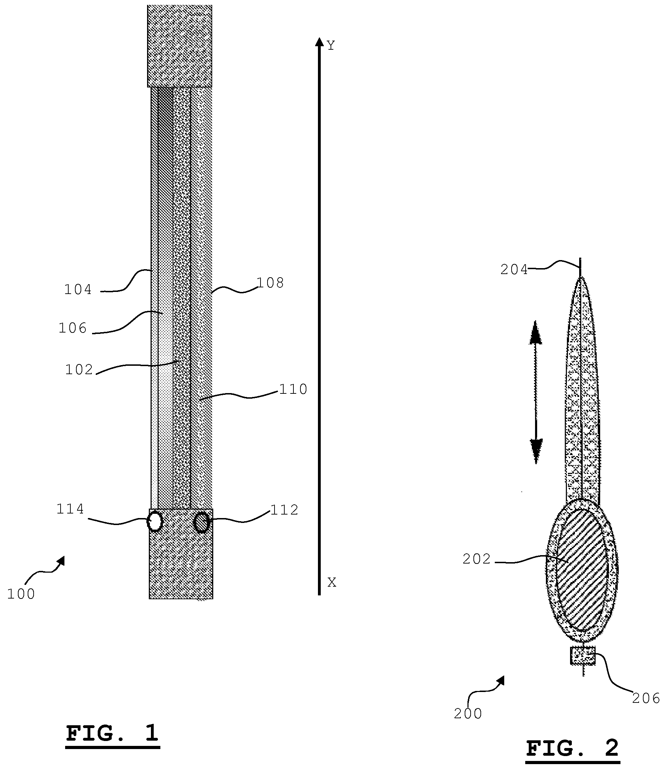

La figure 1 est une représentation schématique d'en exemple de structure multicouche mise en oeuvre dans le dispositif selon l'invention.

La structure multicouche 100 représentée en figure 1 comporte :
- une couche interne 102 faite de matériau élastique, par exemple en néoprène, polyuréthane, latex ...
- une couche externe rigide 104 constituée d'un matériau rigide guidant la propagation des ondes de compression vers l'intérieur du corps, et
- une couche intermédiaire 106 contenant un fluide gélatineux permettant la propagation d'une vague de pression pulsatile progressive et vers le coeur selon la direction naturelle et physiologique du drainage veineux et lymphatique dans la partie sur laquelle est appliquée ladite structure. On considère dans la suite de la description que la direction naturelle et physiologique du drainage veineux et lymphatique est la direction XY représentée sur la figure 1.

La structure multicouche 100 comporte en outre une couche additionnelle 108, comportant un espace 110 en matériel biocompatible, et comportant une paroi en contact du corps microporeuse sur pouvant être remplie d'un produit fluide biocompatible et/ou biologique à travers d'un connecteur 112. Cette partie microporeuse est en contact directe avec la peau. Lors des pulsations, le produit contenu dans cette couche 108 est appliquée sur le corps du sujet par passage au travers de la partie microporeuse.

La couche externe 104 est reliée de manière étanche à des moyens de pulsation (voir figure 2) pour créer des pulsations dans la structure multicouche 100 grâce à un port de connexion 114.

Pour assurer la propagation progression des pulsations le long de la partie du corps sur laquelle la structure multicouche 100 est appliquée, la couche intermédiaire 106 comporte un produit de consistance variable, gélatineux, granuleux ou autre, et distribuant chacune des pulsations progressivement le long de ladite structure multicouche dans la direction XY.

La figure 2 est une représentation schématique d'un exemple de console permettant de créer des pulsations dans la structure multicouche 100 de la figure 1.

La console 200 représentée sur la figure 2 comprend :
- un réservoir pneumatique 202 rempli avec un fluide, par exemple inerte, gazeux ou liquide tel que de l'eau
- un connecteur étanche 204 reliant le réservoir pneumatique 202 à la structure multicouche flexible 100.

Le connecteur étanche 204 est relié directement ou indirectement au port de connexion 114 de la structure multicouche 100.

Le réservoir pneumatique 202 peut être pré-rempli. Le réservoir pneumatique comporte un port 206 permettant d'ajouter, d'enlever ou de remplacer le fluide inerte.

Le réservoir pneumatique 202 peut être directement compressé par le sujet lui-même. En effet, ce réservoir peut être compressé par des pressions exercées par la main du sujet.

Dans un mode de réalisation, le réservoir pneumatique 202 peut être disposé sous une chaussure ou sous le pied du sujet. Des pulsations seront alors crées lors d'une simple marche ou course du sujet.

La console 200 peut en outre comprendre un ou plusieurs moyens de compression du réservoir pneumatique 202 de manière rythmique. Les moyens de compression peuvent être actionnées et contrôlés manuellement ou par un module de contrôle.

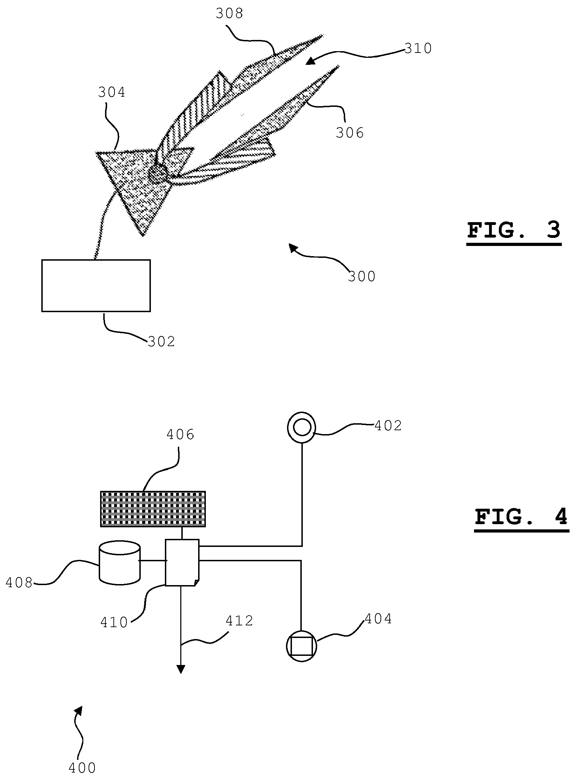

La figure 3 est une représentation schématique d'un exemple de module de compression 300 du réservoir pneumatique 202. Le module de compression 300 comprend une batterie 302 alimentation un ensemble moteur 304 relié à deux plaques 306 et 308 formant entre elles un espace 310 destiné à accueillir le réservoir pneumatique 202. Lorsque l'ensemble moteur est actionné les plaques 306 et 308 sont rapprochées puis écartées de manière rythmique. Chaque rapprochement des plaques 306 et 308 crée une pression et chaque écartement une dépression.

La figure 4 est représentation schématique d'un module de détermination de la fréquence de pulsation.

Le module 400 de détermination des fréquences de pulsation comprend un détecteur du rythme cardiaque 402, un détecteur du rythme respiratoire 404, ainsi des moyens de saisie de données relatives à :
- l'état de santé du sujet, tel que par exemple en bonne santé, risque de défaillance du coeur droit, défaillance du coaur droit, etc...
- la corpulence du sujet, tel que par exemple la taille, le poids, l'âge, etc...
- la partie du corps sur laquelle est appliquée la structure multicouche 100.

Dans l'exemple représenté sur la figure 400, ces moyens de saisie comprennent un écran tactile 406.

Le module 400 peut en outre comprendre une base de données 408 reliée à un programme informatique 410 qui en fonction des données entrées détermine un rythme de pulsation adapté et émet un signal de contrôle 412 permettant de contrôler le module de compression 300.

Nous allons maintenant décrire différents éléments pulsatiles selon l'invention.

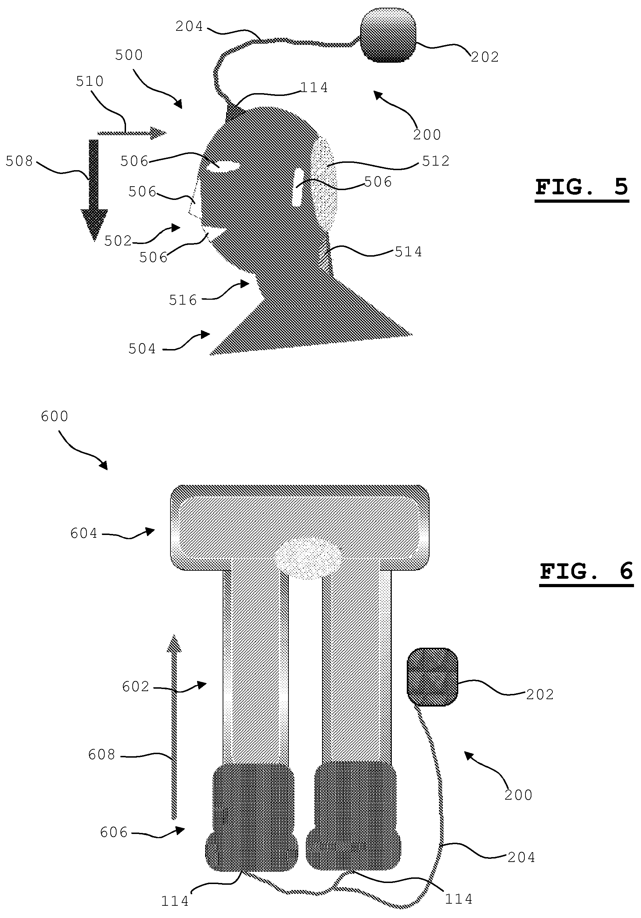

La figure 5 est une représentation schématique d'une cagoule pulsatile 500 selon l'invention. La cagoule pulsatile 500 se compose d'un masque facial 502 et d'un collier 504 réalisées avec la structure multicouche 100 représentée sur la figure 1.

Le masque 502 possède des trous de décompression 506, aux niveaux orbital, buccal, nasal et auriculaire. Un connecteur 114 relie la console pulsatile 200 à une ou plusieurs portes de connexion 114 aménagés sur la couche externe du masque facial 502. Chacune des pulsations réalisées se propage progressivement depuis le port de connexion 114 vers le bas/le coeur selon un axe principe de propagation matérialisé par la flèche 508. Un axe horizontal matérialisé par la flèche 510 représente le trajet des ondes pulsatiles vers le circuit caverneux.

La partie occipitale 512 du masque 502 est peu ou pas gonflable. La partie dorsale 514 au niveau du cou 516 est agencée pour réaliser un massage pulsatile du cou en toute sécurité.

La cagoule 500 sert d'assistance circulatoire pulsatile non-invasive pour traiter le stase veino-lymphatique du visage et du cou. Elle se porte appliquée au visage et au. Ses deux composants, masque 502 et collier 504 fonctionnent en synchronisation rythmique et régulières et en harmonie avec le rythme cardio-respiratoire.

La cagoule présente en outre les fonctions suivantes :
- une fonction principale : la restauration et la réparation des effets secondaires du dysfonctionnement endothélial par application des forces de cisaillement synchronisées avec la diastole, réduisant les congestions lymphatique et veineuse ; et

- une fonction secondaire : l'amélioration hémodynamique de la circulation sanguine du système caverneux agissant sur les maux de tête ou les pertes de mémoires etc.
- amélioration de la circulation cutanée par le monoxyde d'azote augmente et accélère l'absorption et la pénétration de produits cosmétiques existants tels que des produits anti-âge ou soins de la peau.

La couche intérieure du masque 502 peut être modelée sur un masque biologique ou en matériau biocompatible, adapté à la forme du visage et cou. La surface intérieure pourra être microporeuse la diffusion vers la peau de fluides à caractère cosmétiques avec ou sans variation de température des produits ou fluides utilisés, selon les indications.

L'amélioration hémodynamique intervient en deux étapes :
- immédiatement en diminuant la capacitance veineuse stagnante en synchronisation avec la phase diastolique(*). L'augmentation du volume diastolique rythmé, améliore la contractilité ventriculaire, abaisse la post charge pulmonaire et améliore le débit cardiaque global ; et
- a long terme, en améliorant la fonction endothéliale grâce à l'augmentation des forces de cisaillement :
   ▪ diminution de la post charge par excrétion de NO, et
   ▪ stimulation du processus angiogénèse - cardiogénèse myocardique dans le territoire ischémique concerné.

La figure 6 est une représentation schématique d'un pantalon pulsatile 600 selon l'invention.

Le pantalon pulsatile 600 composé d'une partie jambes 602, d'une partie ceinture 604, et d'une partie bottes 606. Dans cette version de l'invention, le pantalon 600 ne comporte pas de couche microporeuse.

Les ondes pulsatiles démarrent au niveau de la partie bottes 606 en provenance de console pulsatile 200. Chacune des pulsations se propagent ensuite vers le coeur selon un axe matérialisé par la flèche 608.

Ce système, outre les fonctions précédemment décrites pour la cagoule pulsatile a une utilisation à la fois réparatrice et prophylactique :
- réparatrice du dysfonctionnement endothéliale grâce aux forces de cisaillement en favorisant l'angiogénèse chez les paraplégiques, ou les malades présentant une fracture de fémur ; et
- prophylactique en empêchant les troubles de coagulation liés au dysfonctionnement endothélial chez les personnes sensibles par exemple au cours d'une longue période d'immobilisation telle que par exemple les vols longue distance, le décubitus prolongé postopératoire et les périodes d'immobilisation lors d'accidents.

Dans une version particulière, le pantalon pulsatile peut comprendre avec une première couche en contact avec la peau à travers les vêtements personnels.

Des modifications de la partie dorsale peuvent être envisagées pour assurer un massage du rachis.

Les communications entre les différents parties (pantalon, ceinture, jambes) seront coordonnées et synchronisées avec le diastole, afin d'éviter un effet de garrot au pli inguinal.

De la même manière peuvent être envisagées une veste pulsatile, des sous vêtements pulsatiles, des bottes pulsatiles, des gants pulsatiles ainsi qu'une combinaison pulsatile complète.

Une combinaison pulsatile complète peut aussi être obtenue par assemblage d'une cagoule, d'une veste, d'un pantalon pulsatile, des gants pulsatiles et des chaussures pulsatiles. Dans ce cas, selon un premier mode de réalisation chaque ensemble pulsatile peut être associé à des moyens de pulsation dédiés. Selon un second mode de réalisation, des moyens de pulsations uniques peuvent être utilisés pour tous les ensembles pulsatiles composant la combinaison pulsatile.

La combinaison pulsatile peut être utilisée à des fins de massage. Une telle combinaison améliore considérablement la *Fatigue** liée au dysfonctionnement endothélial en raison des troubles de l'équilibre apoptose-angiogénèse entraînés par un syndrome inflammatoire, un déficit du système immunitaire, ou une perturbation de l'excrétion du monoxyde d'azote.

Dans une version modifiée la combinaison pulsatile peut comporter une couche additionnelle en contact direct avec la peau, facilitant la diffusion de produits cosmétiques (soins dermatologiques, tonifiants, etc.).

La propagation des impulsions pulsatiles sera synchronisée à partir de plusieurs origines distales telle que les bottes pulsatiles ou des gants pulsatiles.

Chaque ensemble pulsatile pourra être utilisé séparément en fonction des besoins du sujet

En circuit fermé, la combinaison pulsatile pourra être utilisée par des plongeurs, des astronautes, des sportifs et des athlètes permettant d'améliorer physiologiquement les performances immédiates par sécrétion de catécholamines, tout en favorisant à long terme, par l'angiogénèse, le développement de la masse musculaire .

Des forces pulsatile autonomes pourront être obtenues chez les sportifs à partir des gants en serrant les mains ou des bottes au cours du footing.

La combinaison pulsatile peut porter assistance à chaque zone concernée selon les besoin hémodynamiques et biophysiques du sujet, à savoir :
- **Zone 1** dépendante de forces circulatoires accessoires : la combinaison pulsatile améliore l'hémodynamique en réduisant, grâce aux vagues de massage produites, la capacitance veineuse en favorisant le retour du sang vers le coeur au moment de la diastole. L'invention pourra bénéficier à deux groupes d'indications suivantes :
   - Indications pathologiques : insuffisance ventriculaire droite, HTAP chronique, astronautes, plongeurs, varices, paraplégiques, syndrome orthostatique.
   - Indications de confort : Salons de massage, Fitness, Gymnastique, Trajets aériens longue distance ;
- **Zone 2 à 4** dépendante du remplissage diastolique et du rythme : la combinaison permettra d'assurer aux sujets atteints de pathologies cardiaques graves le maintien à long terme de cette fonction physiologique ;
- **Zone** 5 : arbre artériel pulmonaire : la combinaison abaisse les résistances et amélioration la fonction endothéliale.

Chaque dispositif pulsatile selon l'invention est un dispositif d'assistance circulatoire, non-invasif, permettant une réduction progressive de la capacitance veino-lymphatique stagnante. En augmentant la pré charge, le dispositif selon l'invention améliore la contractilité cardiaque ce qui abaisse la post charge entraînant une amélioration hémodynamique globale. À long terme les forces de cisaillement produites par le dispositif pulsatile selon l'invention vont restaurer et préserver la fonction endothéliale. Il transforme le stock sanguin (capacitance veineuse de 64%) et sa masse endothéliale en une porte de secours naturelle en cas de défaillance hémodynamique et circulatoire. Cette méthode plus physiologique, à bas coût, pouvant réduire la morbidité et la mortalité, est applicable chez l'enfant comme chez l'adulte ainsi que chez le sujet animal.

Bien entendu, l'invention n'est pas limitée aux exemples qui viennent d'être décrits et qui illustrent des modes de réalisation particuliers et nullement limitatifs.

## Revendications

1. Dispositif d'assistance circulatoire pulsatile non-invasive (500, 600) favorisant la circulation d'un volume sanguin dans le corps d'un sujet, comprenant :
- une structure multicouche flexible (100) destinée à être appliquée sur au moins une partie du corps dudit sujet, ladite structure comportant une couche interne flexible (102) du côté du corps dudit sujet et une couche externe plus rigide (104),
- des moyens de pulsation (200, 300, 400), reliés à ladite structure multicouche (100) de sorte que l'ensemble structure + moyens de pulsation est étanche, et créant des ondes de pulsations entre lesdites couches internes (102) et externes (104) par l'intermédiaire d'un fluide, dit de pulsation,
ladite structure multicouche comportant des moyens pour guider chacune desdites pulsations progressivement dans le sens du retour veineux vers l'intérieur du corps dudit sujet le long de ladite partie du corps dudit sujet lorsque ladite structure (100) est disposée sur ladite partie du corps dudit sujet, lesdits moyens comprenant un fluide gélatineux et/ou granuleux est contenu dans une couche intermédiaire (106) entre la couche externe (104) et la couche interne (102) réalisant la propagation progressive de chacune des pulsations dans le sens du retour veineux le long de ladite structure (100) et en ce que la structure multicouche (100) comprend une ouverture d'admission (114) du fluide de pulsation provenant des moyens de pulsation (200) entre les couches interne (102) et externe (104).

2. Dispositif selon la revendication 1, **caractérisé en ce qu'**il comprend en outre des moyens pour la détermination d'une fréquence de pulsation en fonction de :
- données relatives au rythme cardiaque,
- données relatives au rythme de respiration,
- données relatives à l'état de santé du sujet, et/ou
- données relatives à la partie à laquelle est appliquée la structure multicouche.

3. Dispositif selon l'une quelconque des revendications précédentes, **caractérisé en ce que** la couche interne (102) comporte, sur au moins une partie, une cavité (110) entre une paroi microporeuse (108) destinée à être mise en contact avec la peau du sujet et une paroi du côté de la couche externe, ladite cavité (110) étant aménagée pour recevoir et/ou conduire un produit destiné à être appliquée sur la peau dudit sujet au travers de ladite paroi microporeuse (108).

4. Dispositif selon la revendication 3, **caractérisé en ce qu'**il comporte une ouverture (112) pour remplir la cavité (110) avec un produit, ladite ouverture (112) étant fermée de manière étanche par des moyens d'obturation lors de l'utilisation.

5. Dispositif selon l'une quelconque des revendications précédentes, **caractérisé en ce que** les moyens de pulsation (200,300) comprennent :
- un réservoir pneumatique (202),
- un moyen de compression (300) dudit réservoir pneumatique (202) de manière rythmique, et
- un connecteur étanche (204) reliant ledit réservoir pneumatique (202) à la structure multicouche flexible (100).

6. Dispositif selon l'une quelconque des revendications 1 à 4, **caractérisé en ce que** les moyens de pulsations comprennent :
- un réservoir pneumatique (202), et
- un connecteur étanche (204) reliant ledit réservoir pneumatique (202) à la structure multicouche flexible (100) ;
l'ensemble réservoir (202), connecteur (204) et structure (100) constituant un circuit fermé pour le fluide de pulsation ; et
ledit réservoir pneumatique (202) étant disposé de sorte qu'il est compressé et décompressé par une force exercée par ledit sujet.

7. Dispositif selon la revendication 6, **caractérisé en ce que** le réservoir, le connecteur et la structure multicouche flexible (100) constituent un ensemble monobloc.

8. Dispositif selon l'une quelconque des revendications précédentes, **caractérisé en ce que** la structure multicouche flexible (100) comprend une cagoule (500) destinée à être disposé sur au moins une partie du visage du sujet.

9. Dispositif selon l'une quelconque des revendications précédentes, **caractérisé en ce que** la structure multicouche flexible comprend pantalon (600).

10. Dispositif selon l'une quelconque des revendications précédentes, **caractérisé en ce que** la structure multicouche flexible comprend une veste.

11. Dispositif selon l'une quelconque des revendications précédentes, **caractérisé en ce que** la structure multicouche flexible comprend un gant.

12. Dispositif selon l'une quelconque des revendications précédentes, **caractérisé en ce que** la structure multicouche flexible comprend une botte ou une chaussette.

13. Ensemble d'assistance circulatoire pulsatile (600) non-invasive couvrant plusieurs parties du corps d'un sujet, ledit ensemble comportant au moins un dispositif selon l'une quelconque des revendications précédentes pour chacune desdites parties.

## Patentansprüche

1. Pulsierende, nicht invasive Vorrichtung zur Unterstützung des Kreislaufes (500, 600), die das Fließen einer Blutmenge im Körper eines Person fördert und folgendes umfasst:
- eine flexible mehrlagige Struktur (100), die dazu bestimmt ist, auf mindestens eine Körperpartie der besagten Person angebracht zu werden, wobei besagte Struktur eine flexible innere Lage (102) zur Körperseite der Person hin und eine steifere äußere Lage aufweist,
- Pulsiermittel (200, 300, 400), die mit besagter mehrlagiger Struktur (100) so verbunden sind, dass die Einheit Struktur + Pulsiermittel dicht ist, und zwischen der inneren Lage (102) und der äußeren Lage (104) mittels eines sogenannten pulsierenden Fluids Pulswellen erzeugen;
wobei besagte mehrlagige Struktur Mittel aufweist, um besagte Pulsierungen nach und nach in Richtung des venösen Rückflusses zum Körperinnern der Person entlang der besagter Körperpartie besagter Person zu leiten, wenn besagte Struktur (100) an besagter Körperpartie der Person angebracht ist, und die besagten Mittel in einer Zwischenlage (106) zwischen der äußeren Lage (104) und der inneren Lage (102) ein gelatineartiges und/oder körniges Fluid enthalten, das die progressive Verbreitung der Pulsierungen in Richtung des venösen Rückflusses entlang der Struktur (100) durchführt, und dadurch dass die mehrlagige Struktur (100) eine Einlassöffnung (114) für das aus den pulsierenden Mitteln (2100) zwischen der inneren Lage (102) und der äußeren Lage (104) kommenden pulsierenden Fluids umfasst.

2. Vorrichtung nach Anspruch 1, **dadurch gekennzeichnet, dass** sie des Weiteren Mittel zur Bestimmung einer Pulsfrequenz unter Berücksichtung folgender Faktoren umfasst:
- der Herzrhythmusdaten
- der Atemrhythmusdaten
- der Daten über den Gesundheitszustand der Person und/oder
- der Daten über die Körperpartie, auf der die mehrlagige Struktur angebracht wird.

3. Vorrichtung nach einem der vorhergehenden Ansprüche, **dadurch gekennzeichnet, dass** die innere Lage (102) an mindestens einem Teil einen Hohlraum (110) zwischen einer mikroporösen Wand (108) aufweist, der dazu bestimmt ist, in Kontakt mit der Haut der Person zu kommen, und einer Wand an der Seite der äußeren Lage, wobei besagter Hohlraum (110) so gestaltet ist, dass er ein Produkt, das über besagte mikroporöse Wand (108) auf die Haut der Person aufgetragen werden soll, aufnehmen und/oder weiterleiten kann.

4. Vorrichtung nach Anspruch 3, **dadurch gekennzeichnet, dass** sie eine Öffnung enthält (112), um den Hohlraum (110) mit einem Produkt zu füllen, wobei besagte Öffnung (112) während der Benutzung durch Verschlussmittel dicht geschlossen bleibt.

5. Vorrichtung nach einem der vorhergehenden Ansprüche, **dadurch gekennzeichnet, dass** die Pulsiermittel (200, 300) folgendes umfassen:
- einen pneumatischen Behälter (202),
- eine Vorrichtung zur rhythmischen Kompression (300) des besagten pneumatischen Behälters (202), und
- einen dichten Verbinder (204), der besagten pneumatischen Behälter (202) mit der flexiblen mehrlagigen Struktur (100) verbindet.

6. Vorrichtung nach einem der Ansprüche 1 bis 4, **dadurch gekennzeichnet, dass** die Pulsiermittel folgendes umfassen:
- einen pneumatischen Behälter (202), und
- einen dichter Verbinder (204), der besagten pneumatischen Behälter (202) mit der flexiblen mehrlagigen Struktur (100) verbindet;
wobei die Einheit bestehend aus Behälter (202), Verbinder (204) und Struktur (100) einen geschlossenen Kreis für das pulsierende Fluid bildet; und
besagter pneumatischer Behälter (202) so angeordnet ist, dass die Kompression und Dekompression des Behälters durch eine von der Person ausgeübte Kraft erfolgt.

7. Vorrichtung nach Anspruch 6, **dadurch gekennzeichnet, dass** der Behälter, der Verbinder und die flexible mehrlagige Struktur (100) eine kompakte Einheit bilden.

8. Vorrichtung nach einem der vorhergehenden Ansprüche, **dadurch gekennzeichnet, dass** die flexible mehrlagige Struktur (100) eine Haube aufweist, die dazu bestimmt ist, auf mindestens einen Teil des Gesichts der Person gelegt zu werden.

9. Vorrichtung nach einem der vorhergehenden Ansprüche, **dadurch gekennzeichnet, dass** die flexible mehrlagige Struktur eine Hose (400) umfasst.

10. Vorrichtung nach einem der vorhergehenden Ansprüche, **dadurch gekennzeichnet, dass** die flexible mehrlagige Struktur eine Jacke umfasst.

11. Vorrichtung nach einem der vorhergehenden Ansprüche, **dadurch gekennzeichnet, dass** die flexible mehrlagige Struktur einen Handschuh umfasst.

12. Vorrichtung nach einem der vorhergehenden Ansprüche, **dadurch gekennzeichnet, dass** die flexible mehrlagige Struktur einen Stiefel oder eine Socke umfasst.

13. Pulsierende, nicht-invasive Einheit zur Unterstützung des Kreislaufes, welche mehrere Körperpartien einer Person bedeckt, wobei die Einheit für jede der Partien mindestens eine Vorrichtung nach einem der vorhergehenden Ansprüche enthält.

## Claims

1. A non-invasive pulsatile circulatory assistance device (500, 600) assisting the circulation of a volume of blood in the body of a subject, comprising
- a flexible multilayer structure (100) intended to be applied to at least part of the body of said subject, said structure comprising a flexible internal layer (102) on the same side as the body of said subject and a more rigid external layer (104),
- pulsation means (200, 300, 400) connected to said multilayer structure (100) so that the assembly consisting of structure + pulsation means is fluidtight, and creating pulsation waves between said internal (102) and external (104) layers by means of a so-called pulsation fluid,
said multilayer structure comprising means for guiding each of said pulsations progressively in the direction of venous return towards the interior of the body of said subject along said part of the body of said subject when said structure (100) is disposed on said part of the body of said subject, said means comprising a gelatinous and/or granular fluid is contained in an intermediate layer (106) between the external layer (104) and the internal layer (102) effecting the progressive propagation of each of the pulsations in the direction of venous return along said structure (100), and in that the multilayer structure (100) comprises an opening (114) for admitting pulsation fluid coming from the pulsation means (200) between the internal (102) and external (104) layers.

2. A device according to claim 1, **characterised in that** it further comprises means for determining a pulsation frequency according to:
- data relating to the heart rate,
- data relating to the respiratory rate,
- data relating to the state of health of the subject, and/or
- data relating to the part to which the multilayer structure is applied.

3. A device according to any one of the preceding claims, **characterised in that** the internal layer (102) comprises, on at least one part, a cavity (110) between a microporous wall (108) intended to be put in contact with the skin of the subject and a wall on the same side as the external layer, said cavity (110) being arranged so as to receive and/or conduct a product intended to be applied to the skin of said subject through said microporous wall (108).

4. A device according to claim 3, **characterised in that** it comprises an opening (112) for filling the cavity (110) with a product, said opening (112) being closed sealingly by obturation means during use.

5. A device according to any one of the preceding claims, **characterised in that** the pulsation means (200, 300) comprise:
- an inflatable reservoir (202),
- a means (300) for compressing said inflatable reservoir (202) rhythmically, and
- a fluidtight connector (204) connecting said inflatable reservoir (202) to the flexible multilayer structure (100).

6. A device according to and one of claims 1 to 4, **characterised in that** the pulsation means comprise:
- an inflatable reservoir (202),
- a fluidtight connector (204) connecting said inflatable reservoir (202) to the flexible multilayer structure (100);
the assembly consisting of reservoir (202), connector (204) and structure (100) constituting a closed circuit for the pulsation fluid; and
said inflatable reservoir (202) being disposed so that it is compressed and decompressed by a force exerted by said subject.

7. A device according to claim 6, **characterised in that** the reservoir, the connector and the flexible multilayer structure (100) constitute a single-piece assembly.

8. A device according to any one of the preceding claims, **characterised in that** the flexible multilayer structure (100) comprises a hood (500) intended to be disposed on at least part of the face of the subject.

9. A device according to any one of the preceding claims, **characterised in that** the flexible multilayer structure comprises a pair of trousers (600).

10. A device according to any one of the preceding claims, **characterised in that** the flexible multilayer structure comprises a jacket.

11. A device according to any one of the preceding claims, **characterised in that** the flexible multilayer structure comprises a glove.

12. A device according to any one of the preceding claims, **characterised in that** the flexible multilayer structure comprises a boot or a sock.

13. A non-invasive pulsatile circulatory assistance assembly (600) covering a plurality of parts of the body of a subject, said assembly comprising at least one device according to any one of the preceding claims for each of said parts.
